Europäisches Patentamt

**European Patent Office**

Office européen des brevets

(11) Publication number: **0 044 441**
A1

# EUROPEAN PATENT APPLICATION

(21) Application number: 81105024.4

(22) Date of filing: 29.06.81

(51) Int. Cl.³: **C 12 P 1/00**
C 12 N 5/00, C 12 N 15/00
//C12R1/91, G01N33/54

(30) Priority: 17.07.80 US 169597

(43) Date of publication of application:
27.01.82 Bulletin 82/4

(84) Designated Contracting States:
CH DE FR GB IT LI

(71) Applicant: SCRIPPS-MILES, INC.
505 South Coast Blvd.
La Jolla, CA 92037(US)

(72) Inventor: Molinaro, Christine Ann
8767 Caminito Abraza
La Jolla CA 92037(CA)

(72) Inventor: Nakamura, Robert Motohara
8841 Nottingham Place
La Jolla CA 92037(CA)

(74) Representative: Senftl, Hannes, Dr. et al,
c/o Bayer AG Zentralbereich Patente.Marken und
Lizenzen
D-5090 Leverkusen 1, Bayerwerk(DE)

(54) Monoclonal antibodies to drugs, methods for their production and somatic cell hybridomas secreting them.

(57) A monoclonal antibody to a drug, usually a haptenic drug of molecular weight between about 100 and 1500 daltons such as gentamicin, produced by somatic cell hybridization. Lymphocytes which produce antibodies to the drug are fused with myeloma cells to form hybridomas, a hydridoma clone which secretes the desired antibody is isolated by conventional procedures, and the secreted monoclonal antibodies are harvested.

EP 0 044 441 A1

## MONOCLONAL ANTIBODIES TO DRUGS, METHODS FOR THEIR PRODUCTION AND SOMATIC CELL HYBRIDOMAS SECRETING THEM

### BACKGROUND OF THE INVENTION

#### 1. *FIELD OF THE INVENTION*

The present invention relates to the production of homogeneous, monospecific anti-drug antibodies raised from somatic cell hybridomas, more specifically, from lymphocyte hybridomas. Such antibodies are conventionally referred to as monoclonal antibodies and are uniquely characterized by chemical and immunological homogeneity. The present invention provides a method of preparing monoclonal antibodies to drugs, the monoclonal antibodies thus produced, and the somatic cell hybridomas formed in the process.

#### 2. *DESCRIPTION OF THE PRIOR ART*

In 1975, Kohler and Milstein reported the production of monoclonal antibodies to sheep red blood cells from hybrid somatic cells formed by fusion of spleen cells from an immunized mouse with murine myeloma cells [*Nature 256*:495(1975)]. Since that time, monoclonal antibodies produced by somatic cell hybridization have been reported for viral antigens [Koprowski *et al, Proc. Natl. Acad. Sci. USA 75*:3938(1978) and U.S. Pat. No. 4,196,265]', tumor-specific antigens

MS-1038

[Koprowski *et al, Proc. Natl. Acad. Sci.* USA 74:2985 (1977] and U.S. Pat. No. 4,172,124], cell surface antigens [Milstein *et al, Cell Biology International Reports* 3:1(1979)], histocompatibility antigens [Galfre *et al, Nature* 266:550(1977)], and a few haptens, such as trinitrophenyl (TNP) [Kohler *et al, Eur. J. Immunol.* 6:511 (1976)], dinitrophenyl (DNP) [Bottcher *et al, Int. Arch. Allergy Appl. Immunol.* 61:248(1980)], and para -azophenyl arsonate [Tung *et al, J. Immunol.* 116:676 (1976)]. Prior to the present invention, there was no report of the production of monoclonal antibodies to pharmacological agents, i.e., drugs. Subsequent to the making of the present invention, monoclonal antibodies to the cardiac glycoside digoxin were reported in an abstract [*Fed. Proc.* 39:928(March 1980)].

For reviews and commentary on the production of monoclonal antibodies by somatic cell hybridization, reference may be made to the following: *Lymphocyte Hybridomas*, ed. Melchers *et al*, Springer-Verlag (New York 1978); *Nature* 266:495(1977); *C & E News*, Jan 1, 1979, p. 15; *C & E News*, April 16, 1979, p. 5; *JAMA* 242:2161(1979); *Med. Lab. Sci.* 36:329(1979); and *Science* 208:692(1980).

## SUMMARY OF THE INVENTION

The present invention provides a method for producing a monoclonal antibody to a drug by somatic cell hydridization techniques. In general, the monoclonal antibody of the present invention is produced by fusing lymphocytes which produce such antibody with myeloma cells to form hybridomas, isolating a hybridoma clone which secretes the desired antibody to the drug of interest, and harvesting the secreted monoclonal antibody. The lymphocytes involved in the hybridization are commonly spleen cells removed from an animal such as a mouse or rat which has been immunized against the

MS-1038

drug by injection of an immunogen conjugate comprising the drug or an analog thereof chemically coupled to an immunogenic carrier material, e.g., protein. Preferably, both the lymphocytes and myeloma cells are of murine origin, with murine myeloma cells deficient in the enzyme hypoxanthine guanine phosphoribosyl transferase being particularly useful.

Formed hybridomas which secrete the desired antibody are isolated, usually by cloning on culture media selective for the fused cell hybridomas and determining by appropriate methods an isolated hybridoma which secretes the desired antibody. The homogeneity of the hybridoma cell line is thereafter preferably assured by subcloning the hybridomas selected for their desirable antibody secretion. Harvesting of the secreted monoclonal antibodies is performed by conventional techniques. Proliferation of antibodies can be accomplished by culturing hybridomas *in vitro* or *in vivo*, e.g., introducing the hybridoma into an animal such as a mouse and removing antibody-rich ascites fluid.

Monoclonal antibodies produced according to the present invention are useful in research and industry, for instance for studying the action of drugs and the interaction of drugs and antibodies therefor, and for assaying liquid media and the like for the drug, e.g., in therapeutic drug monitoring by immunoassay. Competitive binding immunoassays using monoclonal anti-drug antibodies in the detection or determination of a drug has the advantage of minimizing nonspecific background binding of the labeled drug (e.g., drug labeled with a radionucleotide, enzyme, enzyme substrate, fluorescer, chemiluminescer, etc.). Additionally, the use of monoclonal antibodies would maximize the ability to discriminate between the drug under assay and potential cross-

MS-1038

reacting interferants. Furthermore, in the case of homogeneous immunoassays dependent upon steric or allosteric interactions between an antibody and a labeled drug, monoclonal antibodies can be selected to maximize such interaction. On the other hand, in the case of heterogeneous immunoassays wherein such steric or allosteric interactions are disadvantagous, monoclonal antibodies can be selected to minimize such interaction.

DESCRIPTION OF THE PREFERRED EMBODIMENTS

The present invention provides monoclonal antibodies to any drug of choice, i.e., any chemical compound recognized as a pharmacological agent. The drug will be immunogenic, more usually haptenic (incapable of stimulating antibody production unless conjugated to a carrier material), and will vary in molecular weight from about 100 to 5000, more commonly between about 100 and 1500. The chemical structure of the drug will vary widely and will include such classes of compounds as antibiotics, steroids, prostaglandins, lactams, barbiturates, amphetamines, xanthines, and so forth. In terms of pharmacological action, the drug will be an antibiotic, an antiepileptic, an antiasthmatic, an antiarrythmic, an antidepressant, an analgesic, a transquilizer, a cardiovascular agent, a sedative, a bronchodialator, or an antineoplastic agent and so forth. Particular drugs for which monoclonal antibodies can be prepared according to the present invention are gentamicin, tobramycin, amikacin, sisomicin, kanamycin, netilmicin, phenytoin, phenobarbital, primidone, ethosuximide, carbamazepine, valproate, theophylline, propranolol, procainamide, quinidine, amitryptiline, desipramine, disopyramide, doxepin, doxorubicin, nortryptiline, methotrexate, imipramine, lidocaine or digoxin. Other drugs include acetaminophen, chlordia-

MS-1038

**0044441**

zepoxide, chlorpromazine, clonazepam, diazepam, dimetha-dione, ethotoin, flurazepam, glutethimide, haloperidol, isoniazid, mephobarbital, methsuximide, paramethadione, phensuximide, salicylate, thioridazine, tolbutamide and chlorazepate.

Anti-drug antibody-producing lymphocytes may be obtained from various sites, usually the lymph nodes or spleen.  Usually, the selected lymphocytes are spleen cells from an animal which has been immunized against the drug.  In the case of haptenic drugs, immunization is accomplished by injection of the host animal, e.g., mouse, rat, or goat, at one or more of a variety of sites with an immunogen conjugate, normally in mixture with an adjuvant.  Further injections are made at the same or different sites at regular or irregular intervals thereafter until it is evident that anti-drug antibodies are being produced *in vivo*.

Where the drug of interest is haptenic, or only weakly antigenic, the immunogen conjugate used in immunization comprises the drug, or an analog (e.g., derivative or synthetic precursor) which can stimulate the production of antibodies having an acceptable specificity for the drug, chemically linked by covalent bonds to a conventional immunogenic carrier material, most commonly, an immunogenic protein or polypeptide.  For the most part, such proteins and polypeptides will have a molecular weight between 5,000 and 1,000,000 daltons, preferably between 15,000 and 500,000, and more usually between 30,000 and 200,000.  Generally, proteins taken from one species will be immunogenic when introduced to the blood stream of another species.  Particularly useful proteins are albumins, globulins, enzymes, hemocyanins, albuminoids, glutelins, proteins having significant non-proteinaceous constituents, e.g.,

MS-1038

glycoproteins, and the like. The albumins and globulins of molecular weight between 30,000 and 200,000 daltons are particularly preferred. Methods for coupling drugs to such carrier materials are well known in the art [Parker, *Radioimmunoassay of Biologically Active Compounds*, Prentice-Hall (1976)].

Myeloma cells, that is, malignant cells from primary bone marrow tumors, produce immunoglobulins with unknown specificity and have the ability to propagate *in vitro*. In contrast, individual lymphocytes characterized by the secretion of immunoglobulins with known specificity, i.e., antibodies capable of binding a known antigen or hapten, are labile under *in vitro* culturing. A hybridoma, i.e., a hybrid cell formed by fusion of a single lymphocyte with a single myeloma cell, retains the desired immunogical specificity of the lymphocyte parent cell and the desired *in vitro* stability of the myeloma parent cell. Myeloma cells of various animal origin can be used according to the present invention, for example, myeloma cells from mice, rats, or man, however, for reasons of genetic stability it is preferred to fuse lymphocytes and myeloma cells derived from the same animal species, and most preferably, from the same strain of such animal species. Murine lymphocytes and myeloma cells are most commonly used, particularly from the BALB/c strain.

Myeloma cells may be of the secreting or nonsecreting type, the former characterized by secretion of immunoglobulin or fragments thereof from the cell into the surrounding medium. Nonsecreting cell lines are preferred since the resulting hybridomas will feature secretion of only the specific immunoglobulin (e.g., IgG, IgE, IgM, IgA or IgD classes) produced by the parent lymphocyte. Moreover, myeloma cells which

MS-1038

0044441

have been rendered deficient in a particular nucleotide synthetase enzyme are particularly preferred since such a circumstance provides ready means for isolating hybridomas from nonfused lymphocytes and myeloma cells. When grown on a culture medium containing the substrate or substrates for the nucleotide synthetase enzyme in which the myeloma cell is deficient as the only source of nucleotide formation, hybridomas will survive and proliferate since the deficient enzyme will be provided by retained genetic features from the lymphocyte. However, nonfused myeloma cells will die because of enzyme deficiency and nonfused lymphocytes will die ·due to their inherent *in vitro* liability. Preferred myeloma cell lines are those deficient in the enzyme hypoxanthine guanine phosphoribosyl transferase (HGPRT) and unable to survive on culture media containing hypoxanthine, aminopterin, and thymidine for nucleotide synthesis [such media being conventionally referred to as HAT media - *Science 145*:709(1964)]. Particular myeloma cell lines which are useful in the present method are provided in·the following table:

| short name | complete name | IgG secretion | reference |
|---|---|---|---|
| X63 | P3-X63-Ag8 | IgG1, κ | (1) |
| NS-I | P3-NSI/1-Ag4-1 | intracellular κ | (2) |
| Sp2/0 | Sp2/0 Ag14 | nonsecreting κ | (3) |
| - | X63-Ag8:653 | nonsecreting κ | (4) |
| X45 | MPC 11-X45-6TG | IgG2b, κ | (5) |

(1)  *Nature 256*:495(1975)

(2)  *Eur. J. Immunol. 6*:292(1976)

(3)  *Nature 226*:269(1978)

(4)  *J. Immunol. 123*:1548(1979)

(5)  *Cell 8*:405(1976)

MS-1038

Fusing of the lymphocyte and myeloma cells to form hybridomas is accomplished by conventional means, usually by mixing the cells in the presence of a fusing agent such as sendai virus or polyethylene glycol (PEG). PEG is the preferred fusing agent and is available in commercial lots which vary widely in degree of purity and suitability for *in vitro* application. Optimal molecular weights for PEG fusing agents have not been established, with molecular weights between about 1000 and 4000 being conventionally used. In the present study, PEG of molecular weight between about 1000 and 1500 was found to be of particular advantage.

The mechanism of fusion is generally unknown and yields efficiencies of one hybridoma formed for about every $10^4$ normal cells present. The ratio of number of lymphocytes to myeloma cells is usually greater than one, more usually 5 or more. Since an unscreened population of lymphocytes is made available to the myeloma cells for fusion, resulting hybridomas will express a distribution of varying antibody secretions. A hybridoma clone, i.e., a population of cells having a common single cell origin, which secretes the desired antibody is isolated by conventional techniques. A common technique involves the division of the fusion mixture into a multiplicity of diluted volumes in the expectation that the majority of resulting volumes which contain hybridomas will contain no more than one. Preferably, the individual volumes will comprise a selective culture medium, e.g., the HAT medium mentioned above, in which only hybridomas will survive. Aliquots of the separate culture fluids are removed after an appropriate incubation period and subjected to an assay for the desired antibodies. Many methods are commonly used in this screening step, including immunoassay procedures such as radioimmunoassay or noniso-

MS-1038

topic immunoassays of the homogeneous or heterogeneous type. Culture fluids which are positive for the desired antibody are then usually subcloned, such as by further limiting dilution steps, to assure monoclonal isolation and for stability of the desired hybridoma.

The desired monoclonal antibodies can be harvested by several different methods. In one method, the hybridoma clone is cultured *in vitro* for an appropriate length of time and aliquots of the culture fluid drawn off to provide monoclonal antibody-rich fractions. Alternatively, the hybridoma clone is injected into or implanted in a host animal, usually a mouse, resulting in *in vitro* tumor growth and accumulation of large amounts of monoclonal antibody in the ascites fluid which can be appropriately tapped to permit removal of antibody-rich fractions.

Monoclonal antibodies of the present invention will be useful in a wide variety of fields. In particular, the anti-drug monoclonal antibodies are useful as reagents in immunoassays, especially radioimmunoassays and nonisotopic immunoassays of the homogeneous type (see U.S. Patents Nos. 3,817,837; 3,935,074; 3,996,345; 3,998,943 and 4,182,856 and British Patent Specifications Nos. 1,552,607 and 2,023,607) and heterogeneous type (see U.S. Patents Nos. 3,564,090; 4,016,043; Re. 29,169; and 4,201,763). Drug immunoassays have rapidly become an important tool in therapeutic drug monitoring. The availability of monoclonal antibodies to a particular drug of analytical interest provides an antibody reagent for use in immunoassays which is a chemically homogeneous, monospecific reagent, in contrast to the heterogeneity of conventionally obtained antiserum. Hybridomas can be screened to determine the most desirable antibody from the standpoints of binding affinity and cross-reactivity and the selected hybridoma stored for long periods of time, providing a means for producing con-

MS-1038

sistent and well characterized antibodies over a long period of time.

The present invention will now be illustrated, but is not intended to be limited, by the following examples.

Monoclonal Antibody to Gentamicin

A. Preparation of gentamicin immunogen

A gentamicin-hemocyanin immunogen conjugate was prepared by coupling gentamicin to hemocyanin by a conventional carbodiimide method.

B. Immunization

A BALB/c female mouse, 12 weeks old, was rejected intraperitoneally with 100 µg (micrograms) of the gentamicin-hemocyanin immunogen conjugate in Freund's complete adjuvant. Seven weeks later, the mouse was reinjected intraperitoneally with 100 µg of gentamicin-hemocyanin in Freund's complete adjuvant. Ten days after the second injection, the mouse was bled and the serum tested for antibodies to gentamicin. Four days prior to fusion, the mouse was boosted intraperitoneally with 10 µg soluble gentamicin-hemocyanin without adjuvant.

C. Isolation of spleen cells

The mouse was sacrificed by cervical dislocation and the spleen was removed asceptically into a 60 x 15 mm (millimeters) petri dish containing 5 ml (milliliters) serum-free Dulbecco's Modified Essential Medium (DMEM) with high glucose (Flow Laboratories, Inc., Inglewood, CA). The spleen was cut into two fragments. Spleen cells were removed from the splenic capsule with curved forceps. The cells were resuspended with a pipet and then filtered through a funnel lined with nitex screen-ing fabric (110 µ) (Tetko, Monterey Park, CA) into a 17 x 100 mm tube. The tube was centrifuged at 4°C at

MS-1038

350 xg for 7 1/2 minutes. The supernatant was removed and the cells were resuspended by tapping the bottom of the tube. The spleen cells were treated with 5 ml Tris-buffered (10 mM, pH 7.2) ammonium chloride (0.17M) to remove red blood cells.

D. Myeloma cells

SP2/0 Ag 14 [*Nature 226*:269 (1978)] and X63-Ag8.653 [*J. Immunol. 123*:1548(1979)] murine myeloma cell lines were grown in Dubecco's Modified Essential Medium (DME - Flow Laboratories, Inc., Inglewood, CA) supplemented with 10% FBS (fetal bovine serum - Sterile Systems, Logan, UT) and $10^{-4}$ M (molar) 8-azaguanine. Cells in mid-log phase were used as fusing partners.

E. Hybridization procedure

The procedure used for generating hybridomas was a modification of that described in Gefter *et al, Somatic Cell Genetics 3*:231(1977).

Myeloma cells ($2 \times 10^7$ washed twice in serum-free DMEM) were mixed with $1 \times 10^8$ spleen cells from above and the cell mixture spun down. After aspirating all the liquid, the cells were resuspended in a solution of 30% PEG 1000 (polyethylene glycol-MW1000) in serum-free DMEM. The cells remained in this solution for a total of eight minutes. During this eight minute period, the cells were spun at 800 rpm (revolutions per minute) for three minutes. After the eight minutes had elapsed, the fusion reaction was quenched by diluting with 5 ml of serum-free DMEM and the cells were washed once in this medium. The cells were washed a second time in DME supplemented with 10% FBS, NEAA (non-essential amino acid composition from Microbiological Associates, Los Angeles, CA), gentamicin, glutamine, sodium pyruvate, 10% NCTC 109 medium (Microbiological Associates, Los Angeles, CA), bovine insulin (20 I.U./ml) and oxalo-

MS-1038

acetate (1 mM) and finally resuspended in 40 ml of the same medium in 2-T75 flask. The cells were incubated at 37°C in a humidified atmosphere containing 10% $CO_2$.

F.   Cloning procedures

The following day, the cells were pelleted by centrifugation and resuspended in 80 ml of the same medium containing hypoxanthine ($1 \times 10^{-4}$ M), aminopterin ($4 \times 10^{-6}$ M) and thymidine ($1.6 \times 10^{-5}$ M) [HAT medium described in *Science 145*:709(1964)]. The cells were distributed into 96-well, flat-bottomed micro-titer plates (0.1 ml/well). The cells were incubated at 37°C in a humidified atmosphere containing 10% $CO_2$ for one week. At the end of one week, the cultures were fed by adding one drop of the same medium without aminopterin (HT medium) and fed in the same manner at weekly intervals. At the end of the first week, both cell lines generated clones.

The culture fluid of wells showing growth of cells were tested in a radioimmunoassay for antibodies to gentamicin. 96-well Flex-vinyl v-bottom microtiter plates (Cooke Laboratory Products Div., Dynatech Laboratories, Inc., Alexandria, VA) were coated with 100 µl (microliters) of a 1 mg/ml (milligrams per milliliters) solution to gentamicin-BSA at room temperature for two hours or at 4°C overnight. Unattached sites in the wells were saturated with a 10% horse serum PBS (phosphate buffered saline) solution [*Diagnostic Procedures for Viral and Rickettsial Infections*, ed. Venetti *et al*, American Public Health Association (1969) p.156] at room temperature for two hours. To the washed plate was added 25 µl of varying dilutions of mouse anti-gentamicin antiserum (or culture fluid) and the plate was incubated at 4°C for 2-4 hours. After thorough washing of the plate, 50 µl of a 1/5000 dilution of rabbit anti-mouse IgG antiserum was added to each well and the plate was incubated for 2-4 hours at 4°C.

MS-1038

Finally, 50 µl of $^{125}$I-labeled goat anti-rabbit IgG antibody (40,000 cpm/well diluted with 10% horse serum-PBS) was added to the washed wells and the plate was allowed to incubate overnight at 4°C. The wells were thoroughly washed, dried and separated with a hot wire and then individually counted in an automatic gamma counter.

Culture fluids that bound $^{125}$I goat anti-rabbit IgG (giving levels of radiation at least about 2 times greater than background radiation) were retested three days later. Hybridomas that secreted antibodies to gentamicin were subcloned by limiting dilution in a 96-well plate containing 1 x 10$^5$ non-immune BALB/c spleen cells in HT medium. The culture fluids from wells showing growth of cells were tested by a radioimmunoassay for antibody secretion.

Subclones which secreted desired antibodies were expanded *in vitro* and then *in vivo* in Pristane (2,6,10, 14-tetramethylpentadecane) primed mice.


G. Gentamicin assay

In accordance with the Ames TDA Gentamicin test kit (Ames Division, Miles Laboratories, Inc., Elkhart, IN), varying levels of monoclonal antibody to gentamicin were added to 3.0 ml of Bicine buffered β-galactosidase (0.05 µ/ml). At timed intervals, 100 µl of Fluorogenic Gentamicin Reagent was added to each reaction. After incubation for 20 minutes at room temperature the fluorescence was read and gave the following results:

| µl Antibody | Fluorescence Units |
|---|---|
| 0.0 | 93.8 |
| 0.1 | 86.3 |
| 0.2 | 62.1 |
| 0.4 | 13.4 |
| 0.6 | 7.3 |
| 0.8 | 6.7 |
| 1.0 | 5.9 |

MS-1038

- 14 -

The above data shows that the antibody binds the Fluorogenic Gentamicin Reagent and inhibits the enzymically promoted fluorescence.

Fifty μl of varying concentrations of 1:50 diluted gentamicin standards was added to duplicate reaction mixtures containing 0.15 U of β-galactosidase and 0.33 μl of monoclonal antibody in 3.0 ml of Bicine buffer. At timed intervals 100 μl of Fluorogenic Gentamicin Reagent was added to each reaction. After incubation at room temperature for 20 minutes the fluorescence was read and gave the following results:

| Gentamicin (μg/ml) | Average Fluorescence Units |
|---|---|
| 0 | 13.9 |
| 1 | 16.6 |
| 4 | 41.0 |
| 8 | 64.9 |
| 12 | 83.2 |

These data demonstrate that gentamicin monoclonal antibody recognizes both the drug and the fluorogenic reagent to provide a standard curve for use in the assay for gentamicin.

MS-1038

CLAIMS:

1. A monoclonal antibody to a drug.

2. The antibody of claim 1 wherein said drug is a hapten of molecular weight between about 100 and 1500 daltons.

3. The antibody of claim 1 wherein said drug is gentamicin.

4. A somatic cell hybridoma which secretes an antibody to a drug.

5. A method for producing a monoclonal antibody to a drug, comprising the steps of:
   (a) fusing lymphocytes which produce antibodies to said drug with myeloma cells to form hybridomas,
   (b) isolating a hybridoma clone which secretes the desired antibody to said drug, and
   (c) harvesting the secreted monoclonal antibody.

6. The method of claim 5 wherein said drug is a hapten of molecular weight between about 100 and 1500 daltons.

7. The method of claim 5 wherein said drug is an antibiotic, an antiepileptic, an antiasthmatic, an antiarrhythmic, an antidepressant, an analgesic, a tranquilizer, a cardiovascular agent, a sedative, a bronchodialator, or an antineoplastic agent.

MS 1038

8. The method of claim 5 wherein said drug is gentamicin, tobramycin, amikacin, sisomicin, kanamycin, netilmicin, phenytoin, phenobarbital, primidone, ethosuximide, carbamazepine, valproate, theophylline, propranolol, procainamide, quinidine, amitryptiline, desipramine, disopyramide, doxepin, doxorubicin, nortryptiline, methotrexate, imipramine, lidocaine, or digoxin.

9. The method of claim 5 wherein said drug is an antibiotic, a steroid, or a prostaglandin.

10. A method for producing a monoclonal antibody to a haptenic drug, comprising the steps of:
   (a) immunizing an animal with an immunogen conjugate comprising said drug or an analog thereof chemically coupled to an immunogenic carrier material,
   (b) fusing spleen cells from said immunized animal which produce antibodies to said drug with myeloma cells to form hybridomas,
   (c) cloning said hybridomas on culture media selective therefor,
   (d) determining an isolated hybridoma which secretes antibody to said drug,
   (e) subcloning said hybridoma,
   (f) harvesting the secreted monoclonal antibody from said subcloned hybridoma.

MS 1038

## EUROPEAN SEARCH REPORT

**European Patent Office**

Application number

EP 81 10 5024

### DOCUMENTS CONSIDERED TO BE RELEVANT

| Category | Citation of document with indication, where appropriate, of relevant passages | Relevant to claim |
|---|---|---|
| DX | BIO RESEARCH INDEX, vol. 19, ref. 25170<br>M. MUDGETT-HUNTER et al.: "Hybridoma antibodies to the cardiac glycoside digoxin"<br>& Federation Proceedings (USA), vol. 39, no. 3, abstract 3473, 1980<br>  * Title and terms * | 1,2,4-8,10 |
| X | NATURE, vol 285, 12th June 1980, D.S. SECHER et al.: "A monoclonal antibody for large-scale purification of human leukocyte interferon", pages 446-450<br>  * Page 447 * | 1,4,5, 7,10 |
| PX | CHEMICAL ABSTRACTS, vol. 94, no. 25, 22nd June 1981, page 7, abstract 202366a<br>Columbus, Ohio, US<br>B.E. BANG et al.: "Studies of monoclonal and polyclonal antidigoxin antibodies for serum digoxin radioimmunoassay"<br>& Scand. J. Clin. Lab. Invest. 1981, 41(1), 75-8<br>  * The whole abstract * | 1,2,4-8,10 |
| PX | BIO RESEARCH INDEX, vol. 21, ref. 37252<br>J. DOBBINS PLACE et al.: "Production and characterization of mono clonal antibody to gentamycin"<br>                              ./. | 1-10 |

**CLASSIFICATION OF THE APPLICATION (Int. Cl.³)**

```
C 12 P   1/00
C 12 N   5/00
         15/00//
C 12 R   1/91
G 01 N  33/54
```

**TECHNICAL FIELDS SEARCHED (Int. Cl.³)**

```
C 12 P   1/00
C 12 N   5/00
         15/00
A 61 K  39/395
```

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 22-10-1981 | RIJCKEBOSCH |

| | European Patent Office | EUROPEAN SEARCH REPORT | |
|---|---|---|---|

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | & Federation Proceedings (USA), vol. 40, no. 6, page 1594, 1981<br><br>  * Title and terms *<br><br>-- | | |
| PX | BIO RESEARCH INDEX, vol. 21, ref. 35902<br>E. LALLY et al.: "Production and characterization of hybridomas secreting mono clonal antibodies to prostaglandin F-2-alpha"<br><br>& Journal of Dental Research (USA) 1981, vol. 60, no. Spec, Issue A, page 592<br><br>  * Title and terms *<br><br>-- | 1,2,4-7,9,10 | |
| PX | BIO RESEARCH INDEX, vol. 21, ref. 35786<br>A. WHITE et al.: "Development of mono clonal antibodies to steroid hormones"<br><br>& Journal of Endocrinology (GB), 1980, vol. 87, no. 2, pages 7p-8p<br><br>  * Title and terms *<br><br>-- | 1,2,4-7,9,10 | |
| PX | EP - A - 0 014 519 (G. GALFRE et al.)<br><br>  * Page 18, example 4, line 6; page 19, table 2, last example *<br><br>---- | 1,2,4-6,10 | |

| | TECHNICAL FIELDS SEARCHED (Int. Cl.³) |
|---|---|

EPO Form 1503.2  06.78